Europäisches Patentamt

(19) European Patent Office

Office européen des brevets

(11) **EP 0 495 825 B1**

(12) **EUROPÄISCHE PATENTSCHRIFT**

(45) Veröffentlichungstag und Bekanntmachung des
Hinweises auf die Patenterteilung:
**13.03.1996 Patentblatt 1996/11**

(21) Anmeldenummer: **90914901.5**

(22) Anmeldetag: **11.10.1990**

(51) Int. Cl.$^6$: **A61K 31/565**

(86) Internationale Anmeldenummer: **PCT/DE90/00781**

(87) Internationale Veröffentlichungsnummer:
**WO 91/05557 (02.05.1991 Gazette 1991/10)**

(54) **VERWENDUNG VON ANTIGESTAGENEN ZUR HERSTELLUNG VON ARZNEIMITTELN**

USE OF ANTIGESTAGENS IN THE MANUFACTURE OF MEDICAMENTS

UTILISATION D'ANTIGESTAGENES POUR L'OBTENTION DE MEDICAMENTS

(84) Benannte Vertragsstaaten:
**AT BE CH DE DK ES FR GB GR IT LI LU NL SE**

(30) Priorität: **11.10.1989 DE 3934405**

(43) Veröffentlichungstag der Anmeldung:
**29.07.1992 Patentblatt 1992/31**

(73) Patentinhaber: **SCHERING
AKTIENGESELLSCHAFT
D-13342 Berlin (DE)**

(72) Erfinder:
• **MICHNA, Horst
D-1000 Berlin 28 (DE)**
• **SCHNEIDER, Martin
D-1000 Berlin 15 (DE)**
• **EL ETREBY, M. Fathy
Wayne, NJ 07470 (US)**

(56) Entgegenhaltungen:
**EP-A- 0 310 542**

• **Eur. J. Cancer Clin. Oncol., Band 25, Nr. 4, April 1989, Pergamon Press plc, GB; M.R. Schneider et al.: "Antitumor activity of the progesterone antagonists ZK 98.299 and RU 38.486 in the hormone-dependent MXT mammary tumor model of the mouse and the DMBA- and the MNU-induced mammary tumor models of the rat", Seiten 691-701**

• **The Journal of Steroid Biochemistry, Band 34, Nr. 1-6, Mai 1989, Pergamon Press, GB; H. MICHNA et al.: "The antitumor mechanism of progesterone antagonists is a receptor mediated antiproliferative effect by induction of terminal cell death", Seiten 447453**

• **Proceedings of the American Association for Cancer Research, Band 31, März 1990; M.H. SCHNEIDER et al.: "Progesterone antagonists: induction of terminal differentiation and cell cycle blockade in experimental mammary tumors", siehe Seite 220**

• **JNCJ, Band 75, Nr. 3, September 1985; W.L. McGuire et al.: "Emerging impact of flow cytometry in predicting recurrence and survival in breast cancer patients", Seiten 405-410**

• **N. Engl. J. Med., Band 320, Nr. 10, März 1989; G.M. Clark et al.: "Prediction of relapse or survival in patients with node-negative breast cancer by DNA flow cytometry", Seiten 627-633**

• **Cancer, Band 61, Nr. 3, 1988; L.G. Dressler et al.: "DNA flow cytometry and prognostic factors in 1331 frozen breast cancer specimens", Seiten 420-427**

• **W. Forth et al.: "Allgemeine und Spezielle Pharmakologie und Toxikologie", Edition 5, 1987, Bi Wissenschaftsverlag, Mannheim, DE, Seiten 728-737**

**Beschreibung**

Die vorliegende Erfindung betrifft die Verwendung von Antigestagenen (kompetitive Progesteronantagonisten) zur Herstellung von Arzneimitteln zur Behandlung von Mammacarcinomen mit einem erhöhten und als risikoreich eingeschätzten Gehalt an Tumorzellen in der S-Phase des Zellcyclus.

Die bisherige medikamentöse Therapie des Mammacarcinoms mit Antiestrogenen beruht auf der Feststellung, daß das Wachstum und die Vermehrung der entsprechenden Tumorzellen von Estrogenen induziert bzw. abhängig sind.

Es wird zum Beispiel das Antiestrogen Tamoxifen zur palliativen Behandlung des nichtoperablen Mammacarcinoms sowie zur adjuvanten Therapie nach Primärbehandlung des Mammacarcinoms angewandt. Mit Tamoxifen wird die Krankheit jedoch nicht geheilt. Für die Sekundärtherapie werden Gestagene oder Aromatasehemmer verwendet. Aromatasehemmer verhindern die Biosynthese von Estrogenen aus ihren entsprechenden Vorstufen, indem sie das hierfür erforderliche Enzym Aromatase hemmen. In der Praemenopause führen Ovariektomie, Tamoxifen oder LHRH-Analoga (LHRH = Luteinizing hormon releasing hormons) zu vergleichbaren Ansprechraten (Lit. H.T. Mouridsen and R. Paridaens, Eur. J. Cancer Clin. Oncol., 24, S. 99-105, 1988).

In neuerer Zeit wird auch die Verwendung von Antigestagenen im Bereich der Tumortherapie, insbesondere für die Indikation Mammacarcinom diskutiert. Eine erste Phase-II-Studie mit 17β-Hydroxy-11β-(4-dimethylaminophenyl)-17α-(prop-1-inyl)-estra-4,9-dien-3-on an postmenopausalen bzw. ovariektomierten endokrintherapieresistenten Patientinnen mit metastasierendem Mammacarcinom wird von Maudelonde et al. in Hormonal Manipulation of Cancer, Hrsg. J.G.M. Klijn, R. Paridaens und J.A. Folkens in Raven Press, S. 55 (1987) berichtet.

Die gemeinsame Verwendung eines Antigestagens mit einem Antiestrogen für die Behandlung hormonabhängiger Tumoren wurde ebenfalls kürzlich vorgeschlagen (EP-A-0 310 542; Biochem. Biophys. Res. Commun. 1987, 145(2), 706).

Die einzelnen Stadien des Zellcyclus, die von den Tumorzellen durchlaufen werden, bzw. die einzelnen Anteile der Zellen in den verschiedenen Stadien sind von entscheidender Bedeutung für die Entwicklung und den Verlauf der Krankheit sowie für die Chancen einer Therapie. Dem S-Phasengehalt kommt dabei eine besondere Bedeutung zu (Clark, G.M.; Dressler, L.G.; Owens, M.A.; Pounds, G.; Oldaker, T.; Mc Guire, W.L.: Prediction of relapse or survival in patients with node-negative breast cancer by DNA flow cytometry, N. Engl. J. Med. 320: 627-633, 1989 and Mc Guire, W.L.; Dressler, L.G.: Emerging impact of flow cytometry in predicting recurrence and survival in breast cancer patients, JNCI 75(3), 405-410, (1985)).

Aufgabe der vorliegenden Erfindung ist es, ein Arzneimittel anzugeben, welches zur endokrinen Therapie des Mammacarcinoms, dessen Tumorzellen zumindest einen gewissen Gehalt an Tumorzellen in der S-Phase des Zellcyclus aufweisen, geeignet ist.

Diese Aufgabe wird durch die Verwendung von Antigestagenen (kompetitiven Progesteronantagonisten) zur Herstellung eines solchen Arzneimittels gelöst.

Es wurde gefunden, daß Antigestagene (kompetitive Progesteronantagonisten) einen besonders günstigen Einfluß auf die Hemmung des Wachstums von Mammacarcinom-Tumorzellen haben, die zumindest einen gewissen S-Phasengehalt aufweisen. Insbesondere sind solche Mammacarcinome mit Antigestagenen therapeutisch anzugehen, die nach dem Kenntnisstand über die Bedeutung des S-Phasengehaltes im Carcinom als Risikopatienten nach geltender Therapie beurteilt werden. (Clark, G.M. et al., 1989, s.o.). Kompetitive Progesteronantagonisten entfalten sowohl im MXT- als auch im MNU-und DMBA-Tumormodell starke Antitumorwirkung. (MNU = N-Methylnitrosoharnstoff; DMBA = 7,12-Dimethylbenzanthracen; Europ. J. Cancer Clin. Oncol., Vol. 25, No. 4, 691 (1989), M.R. Schneider, H. Michna, Y. Nishino und M.F. El Etreby).

Im Gegensatz sowohl zur Tamoxifen- und Estrogentherapie als auch zur Ovariektomie zeigen die nach Behandlung von Mäusen bzw. Ratten mit Progesteronantagonisten gewonnenen Daten, daß nur Progesteronantagonisten in der Lage sind, die Progression von Tumorzellen in der $G_0G_1$-Phase des Zellcyclus zu blockieren. Dies ergibt sich aus den nachfolgend beschriebenen durchflußcytophotometrischen Messungen an in vivo gewachsenen Tumoren. Mit dieser Blockade geht eine deutliche Abnahme des Anteils der Tumorzellen in der S-Phase einher.

Es war bisher kein hormontherapeutisches Arzneimittel zur Behandlung des Mammacarcinoms bekannt, das eine Absenkung des Gehalts an Tumorzellen in der S-Phase bewirkt.

Der Einfluß von Antigestagenen auf verschiedene Zellcyclusphasen hormonabhängiger Tumoren wird, wie nachfolgend beschrieben, ermittelt. Es werden untersucht:

11β-[(4-N,N-Dimethylamino)-phenyl]-17α-hydroxy-17β-(3-hydroxypropyl)-13α-methyl-4,9(10)-gonadien-3-on (A); EP-A-84730062.1;

11β-(4-Acetylphenyl)-17β-hydroxy-17α-(prop-1-inyl)-4,9(10)-estradien-3-on (B); EP-A-86101548.5;

Tamoxifen und Diethylstilbestrol (DES) dienen als Referenzsubstanzen.

**a) Hormonabhängiges MXT(+)-Mammacarcinom der Maus im Prophylaxemodell**

Als MXT-Tumor wird die von Dr. A.E. Bogden (EG + G Bogden Laboratories, Worcester, MA, USA) als eingefrorene Probe zur Verfügung gestellte MXT-Linie M 3.2 verwendet. Die Inokulation des Tumors auf die Versuchstiere und die Wiederentnahme der gewachsenen Tumoren wird wie in Europ. J. Cancer Clin. Oncol., Vol. 25, No. 4, 691 (1989) von M.R. Schneider, H. Michna, Y. Nishino und M.F. El Etreby beschrieben, durchgeführt. Nach Wiederentnahme werden die Tumoren in Stücke von 2 mm mittleren Durchmessers in MEM (Minimum Essential Medium) 199 geschnitten. Jeweils zwei dieser Stücke werden in BDF1 Mäuse s.c. implantiert. Nach der Transplantation werden die Tiere Gruppen von jeweils 9 bis 10 Tieren zugeordnet. Am nächsten Tag wird mit der Behandlung begonnen. Die zu untersuchenden Verbindungen A und B werden täglich in einer Dosis von je 5 mg/kg/Tier als ölige Lösung (10%ig in Benzylbenzoat) s.c. injiziert oder Ovariektomie wird durchgeführt. Tamoxifen wird in einer Dosis von 5 mg/kg/Tier appliziert. Nach sechswöchiger Behandlung werden die Tiere getötet und gewogen. Nach Entnahme der Tumoren und Bestimmung deren Feuchtgewichte werden die Tumoren in flüssigem Stickstoff eingefroren.

**b) Methylnitrosoharnstoff (MNU) - induziertes Mammacarcinom der SD-Ratte**
Tumoren werden in 50 Tagen alten weiblichen SD-Ratten (Zentralinstitut für Versuchstierzucht, Hannover, FRG) durch einmalige i.v. Injektion von 50 mg MNU in die Schwanzvene induziert. Am 30. Tag nach Induktion wird begonnen, die Tiere nach Tumoren abzutasten (Palpation). Tiere mit mindestens einem Tumor mit einer Tumorfläche von mehr als 150 mm$^2$ werden in Versuchsgruppen eingeteilt. Die Tumoren werden mit den Verbindungen A bzw. B mit einer Dosis von je 20 mg/kg/Tag, mit Tamoxifen mit einer Dosis von 5 mg/kg/Tag und mit DES mit einer Dosis von 0,5 mg/kg/Tag, behandelt.

Die verschiedenen Phasengehalte der Tumoren (S-, $G_0$-$G_1$- und $G_2$M-Phase werden wie von Dressler et al. beschrieben bestimmt (DNA flow cytometry and prognostic factors in 1331 frozen breast cancer specimens, Cancer 61(3), 420, (1988); Evaluation of a modeling system for S-phase estimation in breast cancer by flow cytometry, Canc. Res. 47(20), 5294-5382, (1987)).

Die Ergebnisse gehen aus Tabelle 1 (MXT(+)-Tumor der Maus) und aus Tabelle 2 (MNU-induziertes Mammacarcinom der Ratte) hervor.

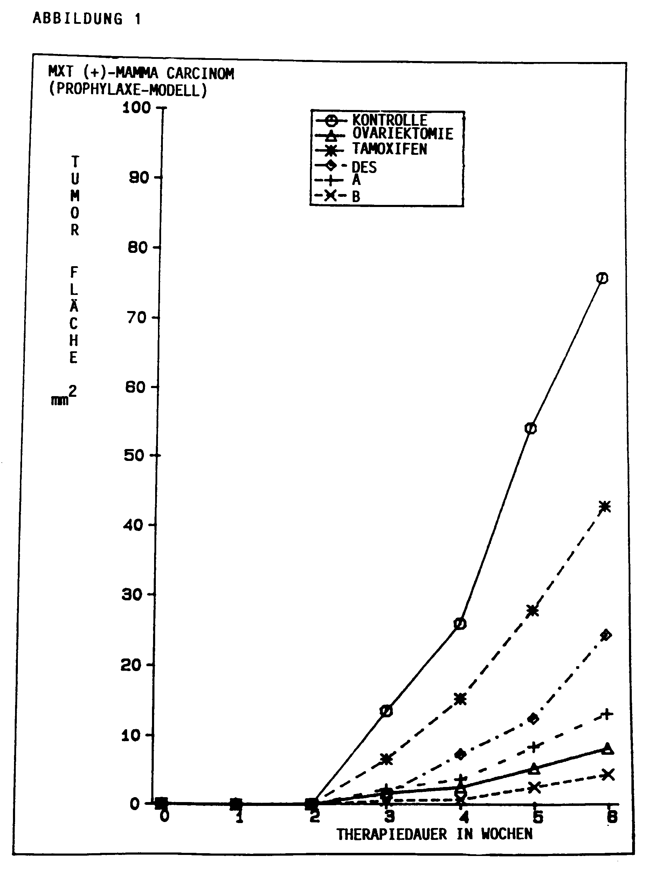

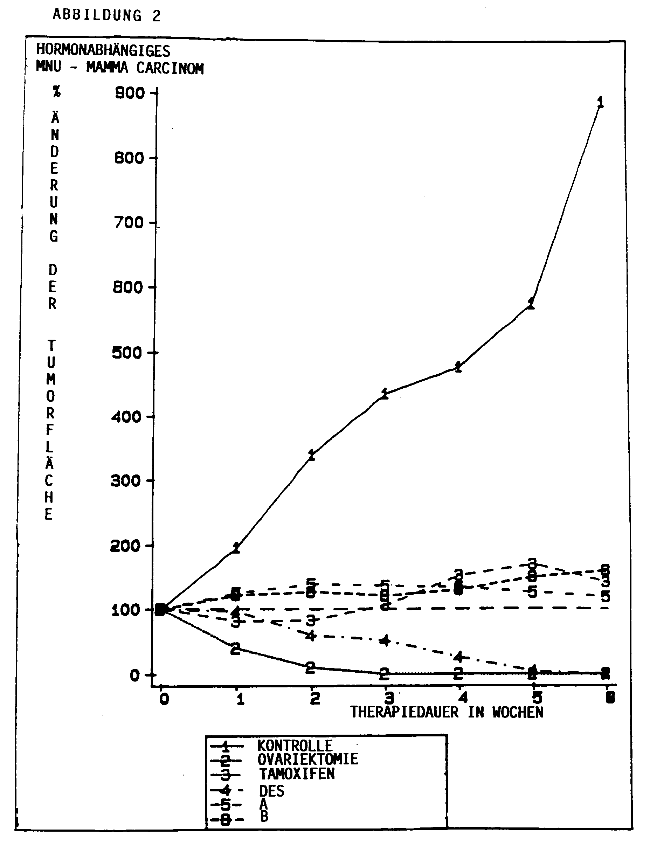

Die Änderung der Tumorflächen im Verlauf der Behandlung mit. Verbindung A bzw. B im Vergleich zur Kontrolle, Ovariektomie und den Referenzverbindungen sind in Abb. 1 (MXT-Tumor) und Abb. 2 (MNU-induzierter Tumor) wiedergegeben.

Von den Tumoren dieser beiden dargestellten Experimente wurden die Untersuchungen zum Zellcyclus durchgeführt (Tabellen 1 und 2).

Die Analysen der Zellcyclusphasen erfolgte für das MXT-Tumormodell nach einer Behandlungsdauer von 4 Wochen (vergl. Abb. 1) und für das MNU-Tumormodell nach einer Therapiezeit von 6 Wochen (vergl. Abb. 2).

TABELLE 1

| MXT-Tumorversuch (Maus) Durchflußcytophotometrische Messungen | | | | | |
|---|---|---|---|---|---|
| Gruppe/Dosis | Tiere pro Gruppe(n) | Zellcyclusphase | | | |
| | | S-Phase | $G_0$-$G_1$ | $G_2M$ | |
| Kontrolle | 3 | 15,22 | 74,21 | 10,57 | |
| | | 21,89 | 71,06 | 7,04 | |
| | | 20,15 | 72,83 | 7,02 | |
| | MW (Mittelwert) | 19,09 | 72,70 | 8,21 | |
| | SD (Standardabweichung) | ± 3,46 | ± 1,58 | ± 2,04 | |
| Tamoxifen | 3 | 16,74 | 69,83 | 13,43 | |
| | | 13,53 | 73,02 | 13,45 | |
| 5 mg/kg | | 16,95 | 73,38 | 9,67 | |
| | MW | 15,74 | 72,08 | 12,18 | |
| | SD | ± 1,92 | ± 1,95 | ± 2,18 | |
| DES | 2 | 14,94 | 77,22 | 7,84 | |
| 2,5 mg/kg | | 14,85 | 77,27 | 7,88 | |
| | MW | 14,90 | 77,25 | 7,86 | |
| | SD | ± 0,06 | ± 0,04 | ± 0,03 | |
| Verbindung (A) | 3 | 0,53 | 97,51 | 1,96 | |
| | | 18,06 | 75,37 | 6,57 | |
| 5 mg/kg | | 18,67 | 75,41 | 5,93 | |
| | MW | 12,42 | 82,76 | 4,82 | |
| | SD | ± 10,30 | ± 12,77 | ± 2,50 | |
| Verbindung (B) | 4 | 0,37 | 98,52 | 1,11 | |
| | | 11,53 | 84,31 | 4,17 | |
| 5 mg/kg | | 14,29 | 79,78 | 5,93 | |
| | | 11,70 | 83,20 | 5,11 | |
| | MW | 9,47* | 86,45* | 4,08 | |
| | SD | ± 6,20 | ± 8,27 | ± 2,11 | |

* = signifikant (p< 0,05) gegen Kontrolle im Kruskal-Wallis-Test

**TABELLE 2**

NMU-Tumorversuch (Ratte)

Durchflußcytophotometrische Messungen

| Gruppe/Dosis | Tiere pro Gruppe(n) | Z e l l c y c l u s p h a s e | | |
|---|---|---|---|---|
| | | S-Phase | $G_0$-$G_1$ | $G_2$M |
| Kontrolle | 6 | 2,52 | 95,43 | 2,05 |
| | | 3,30 | 92,55 | 4,16 |
| ♀ | | 4,36 | 91,82 | 3,83 |
| | | 4,28 | 91,70 | 4,03 |
| | | 4,10 | 92,93 | 2,98 |
| | | 2,74 | 95,20 | 2,06 |
| | MW | 3,55 | 93,27 | 3,19 |
| | SD | ± 0,81 | ± 1,65 | ± 0,97 |
| Ovarektomie | 5 | 4,28 | 93,44 | 2,29 |
| | | 4,16 | 90,61 | 5,22 |
| | | 5,37 | 91,61 | 3,02 |
| | | 2,68 | 94,36 | 2,97 |
| | | 2,23 | 95,25 | 3,52 |
| | MW | 3,74 | 93,05 | 3,40 |
| | SD | ± 1,28 | ± 1,92 | ± 1,11 |
| Tamoxifen | 7 | 2,74 | 95,05 | 2,12 |
| | | 2,58 | 95,01 | 2,40 |
| | | 4,08 | 93,23 | 2,69 |
| 5 mg/kg | | 3,57 | 94,06 | 2,37 |
| | | 7,56 | 87,60 | 4,84 |
| | | 4,28 | 91,70 | 4,03 |
| | | 3,82 | 92,61 | 3,57 |
| | MW | 4,09 | 92,75 | 3,15 |
| | SD | ± 1,66 | ± 2,58 | ±1,02 |
| DES | 3 | 3,89 | 93,59 | 2,53 |
| 0,5 mg/kg | | 8,92 | 83,41 | 7,67 |
| | | 4,02 | 92,25 | 3,73 |
| | MW | 5,61 | 89,75 | 4,64 |
| | SD | ± 2,87 | ± 5,53 | ± 2,69 |

**FORTSETZUNG VON TABELLE 2**

### NMU-Tumorversuch (Ratte)

### Durchflußcytophotometrische Messungen

| Gruppe/Dosis | Tiere pro Gruppe(n) | Z e l l c y c l u s p h a s e | | |
|---|---|---|---|---|
| | | S-Phase | $G_0$-$G_1$ | $G_2$M |
| Verbindung (A) | 5 | 1,91 | 96,17 | 1,92 |
| | | 3,92 | 94,00 | 2,01 |
| 20 mg/kg | | 0 | 97,72 | 2,28 |
| | | 2,21 | 95,63 | 2,16 |
| | | 0 | 98,22 | 1,78 |
| | MW | 1,61* | 96,35 | 2,03* |
| | SD | ± 1,66 | ± 1,69 | ± 0,20 |
| Verbindung (B) | 7 | 0,37 | 98,52 | 1,11 |
| | | 0,94 | 97,00 | 2,05 |
| 20 mg/kg | | 1,04 | 98,21 | 0,74 |
| | | 0,53 | 98,43 | 1,04 |
| | | 0,18 | 98,37 | 1,44 |
| | | 0 | 97,08 | 2,92 |
| | | 0,47 | 98,29 | 1,25 |
| | MW | 0,50* | 97,99* | 1,51* |
| | SD | ± 0,38 | ± 0,65 | ± 0,75 |

\* = signifikant (p < 0,05) gegen Kontrolle im Kruskal-Wallis-Test

Diese bisher für eine Endokrintherapie einzigartige selektive Hemmung der Zellteilung über einen Eingriff in den Zellcyclus sowie des wachsenden Anteils der Masse der Tumorzellen in Verbindung mit der Fähigkeit, die Geschwindigkeit des Absterbens von Zellen aus der proliferativen Population zu beschleunigen, stellt einen hoch-innovativen Mechanismus der Antitumorwirkung von Antigestagenen (kompetitiven Progesteronantagonisten) dar. Die Antigestagene bieten daher einen entscheidenden Vorteil gegenüber allen anderen etablierten endokrinen Behandlungsstrategien bei Brustkrebs, wenn dessen Tumorzellen einen Gehalt an Tumorzellen in der S-Phase des Zellcyclus aufweisen. Die etablierte Therapie der Behandlung des Mammacarcinoms mit Tamoxifen führt dagegen nicht zu einer Verringerung des S-Phasenanteils der Tumorzellen. Der Vorteil der vorgeschlagenen Therapie besteht darin, daß, wenn vor der Therapiefestlegung der S-Phasengehalt des zu behandelnden Mammacarcinoms bestimmt wird, die endokrine Antigestagenbehandlung gezielt und unter Umgehung einer möglicherweise nutzlosen Chemotherapie oder Behandlung mit Tamoxifen ohne Zeitverzögerung bei denjenigen Patientinnen einsetzen kann, deren Tumor(e) Tumorzellen in der S-Phase aufweist(en). Dadurch wird wertvolle Zeit für die Heilung des sich dann noch in einem früheren Stadium befindlichen Tumors gewonnen; gelingt es, den S-Phasenanteil der Tumorzellen zu verringern, so wird dadurch die Überlebenschance der betreffenden Patientin deutlich gesteigert (Mc Guire, W.L. Dressler, L.G., Emerging Impact of flow cytometry in predicting recurrence and survival in breast cancer patients, JNCI, 75(3), 405-410, (1985); Dressler, L.G., Seamer, L.C., Owens, M.A., Clark, G.M., Mc Guire, W.L.: DNA flow cytometry and prognostic factors in 1331 frozen breast cancer specimens; Cancer 61(3), 420-427, 1988).

Hier gezielt einzugreifen, dafür bietet die erfindungsgemäß vorgeschlagene Therapie erstmals eine Chance, indem diejenigen Patientinnen, deren Mammacarcinom einen risikoträchtigen S-Phasengehalt aufweist, unmittelbar mit Antigestagenen (kompetitiven Progesteronantagonisten) behandelt werden. Die erfindungsgemäß behandelbaren Patientinnen stellen etwa 50 % des insgesamt von der Krankheit Mammacarcinom betroffenen Patientinnenkollektivs dar. Es ist erfindungsgemäß auch möglich, zur Herstellung des Arzneimittels zusätzlich zum Antigestagen (zu den Antigestagenen) mindestens noch ein Antiestrogen zu verwenden, da die meisten Mammacarcinome sowohl Progesteronals auch Estrogenrezeptoren aufweisen.

Das Gewichtsverhältnis beider Komponenten kann dabei in weiten Grenzen variiert werden. So können sowohl gleiche Mengen Antigestagen und Antiestrogen als auch ein Überschuß einer der beiden Komponente eingesetzt werden. Antigestagen und Antiestrogen werden gemeinsam, getrennt, gleichzeitig und/oder zeitlich abgestuft (sequential), in einem Gewichtsverhältnis von im wesentlichen 1:50 bis 50:1, vorzugsweise 1:30 bis 30:1, und insbesondere 1:15 bis 15:1 verwendet.

Vorzugsweise können Antigestagen und Antiestrogen kombiniert in einer Dosiseinheit appliziert werden.

Als Antigestagene kommen alle Verbindungen infrage, die eine starke Affinität zum Gestagenrezeptor (Progesteronrezeptor) besitzen und dabei keine eigene gestagene Aktivität zeigen. Als kompetitive Progesteronantagonisten kommen beispielsweise folgende Steroide infrage:

11$\beta$-[(4-N,N-Dimethylamino)-phenyl]-17$\beta$-hydroxy-17$\alpha$-propinyl-4,9(10)-estradien-3-on (RU-38486),

11$\beta$-[(4-N,N-Dimethylamino)-phenyl]-17$\beta$-hydroxy-18-methyl-17$\alpha$-propinyl-4,9(10)-estradien-3-on und

11$\beta$-[(4-N,N-Dimethylamino)-phenyl]-17a$\beta$-hydroxy-17a$\alpha$-propinyl-D-homo-4,9(10),16-estratrien-3-on (alle EP-A 0 057 115);

ferner

11$\beta$-p-Methoxyphenyl-17$\beta$-hydroxy-17$\alpha$-ethinyl-4,9(10)-estradien-3-on (Steroids 37 (1981), 361-382),

11$\beta$-(4-Dimethylaminophenyl)-17$\alpha$-hydroxy-17$\beta$-(3-hydroxypropyl)-13$\alpha$-methyl-4,9-gonadien-3-on (EP-A 0129 499),

11$\beta$-(4-Acetylphenyl)-17$\beta$-hydroxy-17$\alpha$-(prop-1-inyl)-4,9(10)-estradien-3-on (EP-A 0 190 759), sowie

die in der EP-A 0 277 676 beschriebenen 11$\beta$-Aryl-14$\beta$-estradiene und -triene, die 19,11$\beta$-Überbrückten Steroide, die Gegenstand der EP-A 0 283 428 sind, die aus der EP-A 0 289 073 hervorgehenden 11$\beta$-Aryl-6-alkyl (bzw. 6-alkenyl- oder 6-alkinyl)-estradiene und -pregnadiene und die aus der EP-A 0 321 010 bekannten 11$\beta$-Aryl-7-methyl (bzw. 7-ethyl)-estradiene.

Diese Aufzählung ist nicht abschließend; auch andere in den genannten Veröffentlichungen beschriebenen Antigestagene sowie solche aus hier nicht genannten Veröffentlichungen sind geeignet.

Die Antigestagene werden gemäß vorliegender Erfindung in Mengen von 10 mg bis 200 mg eingesetzt; im allgemeinen wird man mit 50 bis 100 mg 11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on pro Tag oder einer biologisch äquivalenten Menge eines anderen Antigestagens auskommen.

Als antiestrogen wirkende Verbindungen kommen Antiestrogene und Aromatasehemmer infrage. Antiestrogene und Aromatasehemmer gemäß vorliegender Erfindung können sowohl von Steroiden abgeleitet oder nicht-steroidale Verbindungen sein.

Unter antiestrogen wirkenden Verbindungen gemäß vorliegender Erfindung sollen aber nur solche Verbindungen verstanden werden, die möglichst selektiv wirken, d.h. die im wesentlichen nur die Wirkung von Estrogen hemmen und/oder deren Konzentration senken. Die Antiestrogene wirken als kompetitive Estrogenantagonisten, indem sie Estrogen vom Rezeptor verdrängen, während Aromatasehemmer die Biosynthese des Estrogens unterdrücken. Verbindungen vom Typ des Aminoglutethimids, 3-alkylierte 3-(4-Aminophenyl)-piperidin-2,6-dione, die außer dem Estrogenspiegel auch auf andere Sexualhormonserumkonzentrationen erniedrigend wirken, sind gemäß vorliegender Erfindung als antiestrogen wirksame Verbindungen nicht geeignet.

Die Antiestrogene können etwa in gleichen Mengen eingesetzt werden wie die bereits im Handel befindlichen Antiestrogene, das heißt die tägliche Dosis beträgt etwa 5 - 100 mg für Tamoxifen oder biologisch äquivalente Mengen eines anderen Antiestrogens.

Als nicht-steroidale Antiestrogene seien beispielsweise genannt:

Tamoxifen = (Z)-2-[p-(1,2-Diphenyl-1-butenyl)-phenoxy]-N,N-dimethyl-äthylamin Nafoxidin = 1-2-[4-(6-Methoxy-2-phenyl-3,4-dihydro-1-naphthyl)-phenoxy]-äthylpyrrolidin, Hydrochlorid und

2-(4-Hydroxyphenyl)-3-methyl-1-[6-(1-pyrrolidinyl)-hexyl]-indol-5-ol und andere in der EP-A-0348341 genannte Aminoalkylindole.

Ferner kommen als steroidale Antiestrogene infrage:

11$\alpha$-Methoxy-17$\alpha$-äthinyl-1,3,5(10)-estratrien-3,17$\beta$-diol,

16$\beta$-Äthylestradiol und

11-(3,17$\beta$-Dihydroxy-1,3,5(10)-estratrien-7$\alpha$-yl)-undecansäure-(N-butyl-N-methyl) amid (EP-A 0 138 504) sowie die in der deutschen Patentanmeldung DE-P 39 25 507.7 beschriebenen Antiestrogene, insbesondere das 11-(3,17-Dihydroxy-14$\alpha$,17$\alpha$-ethano-1,3,5(10)-estratrien-7$\alpha$-yl)-undecansäure-(N-butyl-N-methyl)-amid.

Als Aromatasehemmer sind alle Verbindungen geeignet, die die Bildung von Estrogenen aus ihren Vorstufen hemmen, wie beispielsweise das in der deutschen Offenlegungsschrift 33 22 285 beschriebene 1-Methyl-androsta-1,4-dien-3,17-dion,

das in Journal of Clinical Endocrinology and Metabolism. 49, 672 (1979) beschriebene Testolacton (17a-Oxa-D-homoandrost-1,4-dien-3,17-dion), die in "Endocrinology" 1973, Vol. 92, No. 3, Seite 874 beschriebenen Verbindungen Androsta-4,6-dien-3,17-dion,

Androsta-4,6-dien-17β-ol-3-on-acetat,

Androsta-1,4,6-trien-3,17-dion,

4-Androsten-19-chlor-3,17-dion,

4-Androsten-3,6,17-trion,

die in der deutschen Offenlegungsschrift 31 24 780 beschriebenen 19-alkinylierten Steroide,

die in der deutschen Offenlegungsschrift 31 24 719 beschriebenen 10-(1,2-Propadienyl)-steroide,

die in der europäischen Patentanmeldung EP-A 100 566 beschriebenen 19-Thioandrostanderivate,

das in "Endocrinology" 1977, Vol. 100, No. 6, Seite 1684 und der US-Patentschrift 4,235,893 beschriebene 4-Androsten-4-ol-3,17-dion und dessen Ester, die in der deutschen Offenlegungsschrift 35 39 244 beschriebenen 1-Methyl-15α-alkyl-androsta-1,4-dien-3,17-dione,

die in der deutschen Offenlegungsschrift 36 44 358 beschriebenen 10β-Alkinyl-4,9(11)-estradien-derivate

und das in der europäischen Patentanmeldung 0250262 beschriebene 1,2β-Methylen-6-methylen-4-androsten-3,17-dion.

Als nicht-steroidale Aromatasehemmer seien beispielsweise das [4-(5,6,7,8-Tetrahydroimidazo[1,5a]-pyridin-5-yl)benzonitril-monohydrochlorid] (Cancer Res., 48, S. 834-838, 1988) und die aus der EP-A-0293978 hervorgehenden Triazolderivate erwähnt.

Die Dosierung liegt bei 1 - 1000 mg 1-Methyl-androsta-1,4-dien-3,17-dion pro Tag oder biologisch äquivalenten Dosen von anderen Aromatasehemmern.

Antigestagen- und antiestrogenwirksame Verbindungen können zum Beispiel lokal, topisch, subcutan, enteral oder parenteral appliziert werden.

Für die enterale Applikation kommen insbesondere Tabletten, Dragees, Kapseln, Pillen, Suspensionen oder Lösungen infrage, die in üblicher Weise mit den in der Galenik üblichen Zusätzen und Trägersubstanzen hergestellt werden können. Für die lokale oder topische Anwendung kommen beispielsweise Vaginalzäpfchen oder transdermale Systeme wie Hautpflaster infrage. Die bevorzugte subcutane Injektion wird mit einer öligen Lösung der betreffenden Komponente(n) vorgenommen.

Eine Antigestagen-Dosiseinheit enthält etwa 10 - 200 mg 11β-[(4-N,N-Dimethylamino)-phenyl]-17α-hydroxy-17β-(3-hydroxypropyl)-13α-methyl-4,9(10)-gonadien-3-on oder eine biologisch äquivalente Menge eines anderen Antigestagens.

Eine Antiestrogen-Dosiseinheit enthält 1 - 100 mg Tamoxifen oder 10 - 200 mg 1-Methyl-androsta-1,4-dien-3,17-dion oder eine biologisch äquivalente Menge einer anderen antiöstrogen wirksamen Verbindung.

**Beispiel 1**

10,0 mg 11β-[(4-N,N-Dimethylamino)-phenyl]-17α-hydroxy-17β-(3-hydroxypropyl)-13α-methyl-4,9-gonadien-3-on

140,5 mg Laktose

69,5 mg Maisstärke

2,5 mg Polyvinylpyrrolidon 25

2,0 mg Aerosil

0,5 mg Magnesiumstearat

225,0 mg Gesamtgewicht der Tablette

**Beispiel 2**

50,0 mg 1-Methyl-androsta-1,4-dien-3,17-dion

115,0 mg Laktose

50,0 mg Maisstärke

2,5 mg Poly-N-Vinylpyrrolidon 25

2,0 mg Aerosil

0,5 mg Magnesiumstearat

220,0 mg Gesamtgewicht der Tablette

**Patentansprüche**

1. Verwendung von Antigestagenen (kompetitive Progesteronantagonisten) zur Herstellung von Arzneimitteln zur Behandlung von Mammacarcinomen mit einem erhöhten und als risikoreich eingeschätzten Gehalt an Tumorzellen in der S-Phase des Zellcyclus.

2. Verwendung von Antigestagenen nach Anspruch 1 zusätzlich mit mindestens einem Antiestrogen.

**3.** Verwendung von 11β-[(4-N,N-Dimethylamino)-phenyl]-17α-hydroxy-17β-(3-hydroxypropyl)-13α-methyl-4,9(10)-gonadien-3-on nach Anspruch 1.

**4.** Verwendung von 11β-(4-Acetylphenyl)-17β-hydroxy-17α-(prop-1-inyl)-4,9(10)estradien-3-on nach Anspruch 1.

**5.** Verwendung von Tamoxifen nach Anspruch 2.

**6.** Verwendung von 11-(3,17β-Dihydroxy-1,3,5(10)-estratrein-7α-yl)-undecansäure-(N-butyl-N-methyl)-amid nach Anspruch 2.

**7.** Verwendung von 11-(3,17-Dihydroxy-14α,17α-ethano-1,3,5(10)-estratrien-7α-yl)-undecansäure-(N-butyl-N-methyl)-amid nach Anspruch 2.

## Claims

**1.** Use of antigestagens (competitive progesterone antagonists) for the production of medicaments for the treatment of mammary carcinomas having an increased content, considered to be high-risk, of tumour cells in the S-phase of the cell cycle.

**2.** Use of antigestagens according to claim 1 additionally with at least one anti-oestrogen.

**3.** Use of 11β-[(4-N,N-dimethylamino)-phenyl]-17α-hydroxy-17β-(3-hydroxypropyl)-13α-methyl-4,9(10)-gonadien-3-one according to claim 1.

**4.** Use of 11β-(4-acetylphenyl)-17β-hydroxy-17α-(prop-1-ynyl)-4,9(10)-oestradien-3-one according to claim 1.

**5.** Use of tamoxifen according to claim 2.

**6.** Use of 11-(3,17β-dihydroxy-1,3,5(10)-oestratrien-7α-yl)-undecanoic acid (N-butyl-N-methyl)-amide according to claim 2.

**7.** Use of 11-(3,17-dihydroxy-14α,17α-ethano-1,3,5(10)-oestratrien-7α-yl)-undecanoic acid (N-butyl-N-methyl)-amide according to claim 2.

## Revendications

**1.** Utilisation d'antigestagènes (antagonistes compétitifs de la progestérone) pour préparer des médicaments pour le traitement de cancer du sein avec une teneur élevée en cellules tumorales en phase S du cycle cellulaire, considérées comme risque pour le patient.

**2.** Utilisation d'antigestagènes selon la revendication 1, avec en plus au moins un anti-oestrogène.

**3.** Utilisation de la 11β-[(4-N,N-diméthylamino)-phényl]-17α-hydroxy-17β-(3-hydroxypropyl)-13α-méthyl-4,9(10)-gonadiène-3-one selon la revendication 1.

**4.** Utilisation de la 11β-(4-acétylphényl)-17β-hydroxy-17α-(prop-1-inyl)-4,9(10)-estradiène-3-one selon la revendication 1.

**5.** Utilisation du tamoxifène selon la revendication 2.

**6.** Utilisation du (N-butyl-N-méthyl)amide de l'acide 11-(3,17β-dihydroxy-1,3,5(10)-estratriène-7α-yl)-undécanoïque selon la revendication 2.

**7.** Utilisation du (N-butyl-N-méthyl)-amide de l'acide 11-(3,17-dihydroxy-14α,17α-éthano-1,3,5(10)-estratriène-7α-yl)-undécanoïque selon la revendication 2.

ABBILDUNG 1

MXT (+)-MAMMA CARCINOM
(PROPHYLAXE-MODELL)

Legend:
- KONTROLLE
- OVARIEKTOMIE
- TAMOXIFEN
- DES
- A
- B

Y-axis: TUMOR FLÄCHE $mm^2$ (0 to 100)

X-axis: THERAPIEDAUER IN WOCHEN (0 to 6)

ABBILDUNG 2